# EUROPEAN PATENT SPECIFICATION

(11) **EP 0 941 117 B1**
(45) Date of publication and mention of the grant of the patent: **28.08.2002**
(21) Application number: 97950137.6
(22) Date of filing: 04.11.1997
(51) Int. Cl.: A61K 39/10, A61K 39/05, A61K 39/08

(54) **ACELLULAR PERTUSSIS VACCINE WITH DIPHTHRIAE- AND TETANUS-TOXOIDS**
AZELLULÄRER PERTUSSIS-IMPFSTOFF, DER EIN DIPHTHERIE-TOXOID UND EIN TETANUS-TOXOID ENTHÄLT
VACCIN ACELLULAIRE ANTICOQUELUCHEUX A ANATOXINES DIPHTERIQUE ET TETANIQUE

(30) Priority: 07.11.1996 GB 9623233
(43) Date of publication of application: 15.09.1999
(60) Divisional application: 02075821.5
(73) Proprietor: SMITHKLINE BEECHAM BIOLOGICALS S.A., 1330 Rixensart (BE)
(72) Inventor: FLORENT, Patrick, SmithKline Beecham Biologicals, B-1330 Rixensart (BE); STEPHENNE, Jean, SmithKline Beecham Biologicals SA, B-1330 Rixensart (BE); VANDECASSERIE, Christian, SmithKline Beecham Biol., 1330 Rixensart (BE)
(74) Representative: Tyrrell, Arthur William Russell, Dr.
(86) International application number: EP9706180
(87) International publication number: WO98019702

(56) References cited:
- WO-A-93/24148
- EDWARDS K M ET AL: "Comparison of 13 acellular pertussis vaccines: Overview and serologic response." PEDIATRICS 96 (3 PART 2). 1995. 548-557. ISSN: 0031-4005, XP002058253
- PODDA A ET AL: "Comparative study of a whole-cell pertussis vaccine and a recombinant acellular pertussis vaccine." JOURNAL OF PEDIATRICS 124 (6). 1994. 921-926. ISSN: 0022-3476, XP002058254
- GRECO D ET AL: "A controlled trial of two acellular vaccines and one whole-cell vaccine against pertussis. Progetto Pertosse Working Group" NEW ENGLAND JOURNAL OF MEDICINE, vol. 334, no. 6, 8 February 1996, pages 341-348, XP002058255

## Description

The present invention relates to new vaccine formulations, comprising a low dose of the 69kda outer membrane protein of Bordetella pertussis (hereinafter termed '69K' or '69K antigen' or pertactin, disclosed in European Patent 0 162 639. Recombinant 69K (P69) has been described by N F Fairweather et al, Symposium On Pertussis (Bethesda), 26-28 September 1990). The invention in particular relates to a vaccine comprising more than one antigen, especially a multivalent vaccine, that is: a vaccine for the amelioration or treatment of more than one disease state, in which a low dose of 69K is present. The present invention also relates to the production and use of such vaccines in medicine.

It is known that 69K is an important component of acellular pertussis vaccines (Pa vaccines) for the effective prevention of pertussis.
A study on the dose responses of 5 acellular pertussis vaccines in healthy adults was published by the US National Institutes of Health (NIH) in May 1996 by Keitel, W. et al.
69K-containing vaccines including 'trivalent' vaccines comprising antigens against diphtheria (D), tetanus (T) and pertussis (Pa) have been described in clinical trials conducted in Italy and Sweden and are marketed [for example under the Trade Mark INFANRIX-DTPa (SmithKline Beecham Biologicals)]. Typically the pertussis component of such vaccines comprises pertussis toxin (PT), filamentous haemagglutinin (FHA) and 69K. In INFANRIX-DTPa (Trade Mark) the amounts of D:T:PT:FHA:69K are typically 25 Lf: 10Lf: 25µg:25µg:8µg per 0.5 ml dose of bulk vaccine.

Other multivalent vaccines comprising 69K are also known, for example vaccines comprising an antigen against hepatitis B and antigens against diphtheria, tetanus and pertussis (Hep B, DTPa) were described in WO 93/24148. In such formulations the quantity of D.T:PT:FHA:69K was given as 25 Lf: 10Lf: 25µg:25µg:8µg per 0.5 ml dose of bulk vaccine.

A comparison of 13 diphtheria-tetanus-acellular pertussis vaccines is reported in "Pediatrics, 96 (3), part 2 of 2, supplement, 548-57".

It has now been found that a diphtheria, tetanus and pertussis vaccine containing a low dose of each of diphtheria toxoid (D), tetanus toxoid (T), PT, FHA and 69K (herein such a low dose formulation is abbreviated to a dtpa vaccine) maintains an ability to prevent pertussis (and is highly effective in this respect) while having the advantage of showing exceptionally low reactogenicity Within the limits described herein such vaccines are safe when administered to human subjects and induce rapid protection against infection. In combination vaccines comprising dtpa and other antigens it has been found that there is no interference, i.e. such vaccines show no loss of immunogenicity, and are effective when administered to humans.

In particular the vaccines of this invention are suitable for administration to children as a booster following prior administration of one or more (typically up to about 3) doses of a vaccine comprising a higher dose of D, T, and Pa antigens such as the INFANRIX-DTPa vaccine described above (D:T:PT:FHA:69K = 25 Lf: 10Lf: 25µg:25µg:8µg per 0.5 ml dose of bulk vaccine). The vaccines of this invention are also of great value for administration to adults and adolescents.

Accordingly the present invention provides a vaccine composition comprising a low dose of each of the antigens D, T, PT, FHA and 69K (i.e. a dtpa vaccine composition). It will be understood that the vaccine composition of the invention is formulated with a suitable carrier or adjuvant.

By dtpa vaccine or dtpa vaccine composition is meant a dose wherein the concentration of D per 0.5 ml dose of bulk vaccine is 1-4 Lf, more preferably 2 Lf; the concentration of T per 0.5 ml dose of bulk vaccine is 2.5 - 7.5 Lf, more preferably 5Lf: the concentration of PT per 0.5 ml dose of bulk vaccine is 2-10 µg, more preferably 8µg; the concentration of FHA per 0.5 ml dose of bulk vaccine is 2-10 µg, more preferably 8µg; and the concentration of 69K is in the range 0.5µg to 3 µg per 0.5 ml dose of bulk vaccine. More preferably the concentration of 69K in the vaccine is 2.5µg pertactin per 0.5 ml dose of bulk vaccine.

In a preferred aspect the vaccine compositions of the invention comprise PT (8µg), FHA (8ug), 69K (2.5µg) per 0.5 ml dose of bulk vaccine.

In an especially preferred aspect the vaccine compositions of the invention comprise PT (8µg), FHA (8µg), 69K (2.5µg), D (2 Lf) and T (5 Lf) per 0.5 ml dose of bulk vaccine. This was used in the 'dtpa 005' study reported below.

The following dtpa tricomponent vaccine composition was prepared:
PT 8 µg
FHA 8 µg
69K 2.5 µg
diphtheria toxoid >= 2 IU
tetanus toxoid >= 20 IU
Al salts 0.3 mg
phenoxyethanol 2.5 mg
This was used in the study in adolescents known as 'dtpa 003', results of which are given below.

Optionally the PT component may be recombinant (for example as described in European Patent Applications EP 0 306 318, EP 0 322 533, EP 0 396 964, EP 0 322 115 and EP 0 275 689) or the PT component may be toxoided, for example as described in EP 0 515 415. See also EP 0 427 462 and WO 91/12020 for the preparation of pertussis antigens.

In a further aspect the invention provides a vaccine composition comprising dtpa (as hereinabove defined) in combination with one or more additional antigens, particularly an antigen against hepatitis B (Hep B) (i.e. a Hep B - dtpa vaccine). Such multivalent vaccines are generally as described in WO 93/24148 except that a low dose of each of D, T, PT, FHA and 69K as hereinabove defined is used in the formulation. As described in WO 93/24148 the hepatitis B antigen is preferably hepatitis B surface antigen.

The dose of hepatitis B surface antigen will normally be in the range 5-20µg per 0.5 ml dose of bulk vaccine.

The preparation of Hepatitis B surface antigen (HBsAg) is well documented. See for example, Harford et al in Develop. Biol. Standard 54, page 125 (1983), Gregg et al in Biotechnology, 5, page 479 (1987), EP-A- 0 226 846, and EP-A-0 299 108.

As used herein the expression 'Hepatitis B surface antigen' or 'HBsAg' includes any HBsAg antigen or fragment thereof displaying the antigenicity of HBV surface antigen. It will be understood that in addition to the 226 amino acid sequence of the HBsAg S antigen (see Tiollais et al, Nature, 317, 489 (1985) and references therein) HBsAg as herein described may, if desired, contain all or part of a pre-S sequence as described in the above references and in EP-A- 0 278 940. HBsAg as herein described can also refer to variants, for example the 'escape mutant' described in WO 91/14703. In a further aspect the HBsAg may comprise a protein described as L* in European Patent Application Number 0 414 374, that is to say a protein, the amino acid sequence of which consists of parts of the amino acid sequence of the hepatitis B virus large (L) protein (ad or ay subtype), characterised in that the amino acid sequence of the protein consists of either:
(a) residues 12 - 52, followed by residues 133 - 145, followed by residues 175 - 400 of the said L protein; or
(b) residue 12, followed by residues 14 - 52, followed by residues 133 - 145, followed by residues 175 - 400 of the said L protein.

HBsAg may also refer to polypeptides described in EP 0 198 474 or EP 0 304 578.

Normally the HBsAg will be in particle form. It may comprise S protein alone or may be as composite particles, for example (L*,S) wherein L* is as defined above and S denotes the S-protein of hepatitis B surface antigen.

Preferably the HBsAg will be adsorbed on aluminium phosphate as described in WO93/24148. Other antigens may be adsorbed onto aluminium phosphate or aluminium hydroxide but in some cases satisfactory results will be obtained only if the other antigen is adsorbed on aluminium phosphate.

Other antigens may included in vaccines of the invention to provide other multivalent vaccines for paediatric, adolescent or adult use. Suitable said other antigens may, for example, comprise those known in the art to provide protection against Haemophilus influenzae b (Hib) and/or polio (IPV) and/or hepatitis A, as described in WO 93/24148.

Suitable components for use in such vaccines are already commercially available and details may be obtained from the World Health Organisation. For example the IPV component may be the Salk inactivated polio vaccine. The component affording protection against Hepatitis A is preferably the product known as 'Havrix' (SmithKline Beecham Biologicals) which is a killed attenuated vaccine derived from the HM-175 strain of HAV [see 'Inactivated Candidate Vaccines for Hepatitis A' by F.E. Andre, A Hepburn and E.D'Hondt, Prog Med Virol. Vol 37, pages 72-95 (1990) and the product monograph 'Havrix' published by SmithKline Beecham Biologicals (1991)]. Conveniently, the Hepatitis B component may comprise the 'S' antigen as already present in the commercial vaccine 'Engerix-B' (SmithKline Beecham Biologicals).

The amount of antigen in each vaccine dose is selected as an amount which induces an immunoprotective response without significant, adverse side effects in typical vaccinees. Such amount will vary depending on which specific immunogens are employed. Generally it is expected that each dose will comprise 15-50ug of total immunogen. Booster injections may be given. Booster injections of vaccines according to the invention in subjects primed with a vaccine comprising whole cell pertussis (Pw) are as efficacious as in subjects primed with a vaccine comprising acellular pertussis (Pa).

In general the vaccine formulations of any aspect of the invention can be prepared as follows. The required components are adsorbed onto a suitable adjuvant, most especially aluminium hydroxide or aluminium phosphate. After allowing time for complete and stable adsorption of the respective components, the different components can be combined under appropriate conditions.

In a preferred aspect of preparing a combined Hepatitis B-containing vaccine composition according to the invention there is provided a method which involves mixing aluminium phosphate-adsorbed HBsAg with one or more aluminium hydroxide or aluminium phosphate-adsorbed antigens.

The following examples illustrate the invention:

### Example I: Preparation of a dtpa vaccine formulation and study in adults

The dtpa vaccine according to the invention was prepared by standard methodology in accordance with the procedure for formulating a higher dose DTPa vaccine described in WO 93/24148.

Results obtained with the vaccine in a clinical study in adults coded 'dtpa 005' are shown in the appended figures, in which dtpa is used to signify a vaccine according to the invention and PRN is an abbreviation for pertactin (69K). The abbreviation dT is used to signify the standard diphtheria-tetanus vaccine obtainable from Behringwerke comprising a low dose of diphtheria toxoid and a higher dose of tetanus toxoid.

An open, randomised single dose study was carried out in adults aged 18-45 in Belgium using dtpa formulated as described above in 60 subjects as compared with dT (Behring) in 60 subjects.

For the dtpa group 28 females were studied (mean age 33 years) and 32 males were studied (mean age 33 years). For the dT group 32 females were studed (mean age 34 years) and 28 males were studied (mean age 35 years).

Excluded were those who had had a diphtheria, tetanus or pertussis vaccination within the last 10 years or pertussis disease within the last 5 years.

Sera were obtained pre and I month post vaccination with a solicited follow-up after 15 days and an unsolicited follow-up after 30 days.

Reactogenicity data, antibody responses, anti-PT results, anti-FHA results and anti-PRN (pertactin) results are shown in the appended figures.

In the appended figures, results are also shown for the monovalent pa vaccine used in the NIH (AAPT) studies reported by Keitel et al. References to SmithKline Beecham's (SB's) High, Medium (SB-Med) and Low formulations contain, respectively, PT: (25µg), FHA (25µg) and 69K (8µg) (High); PT (8µg), FHA (8µg) and 69K (2.5µg) (Medium); and PT (2.5µg), FHA (2.5µg) and 69K (0.8µg) (Low).

The abbreviation 'µg' stands for micrograms.

It may be seen that a dtpa vaccine according to the invention was safe and immunogenic in adults. Furthermore immune responses to candidate vaccines of the invention in adults appeared to be at least several times higher than observed for known higher dose DTPa vaccines previously administered to US, German and Italian infants.

### Example 2: Study in adolescents ('dtpa 003')

An open, randomised single dose study was carried out in adolescents aged 10-12 in Finland using dtpa formulated as described above in 120 subjects as compared with dT (Finland) in 120 subjects. The same lot of dtpa vaccine of the invention was used in both the dtpa 005 and dtpa 003 studies.

The commercially available (Finland) dT contained: diphtheria toxoid >= 4 IU tetanus toxoid >= 40 IU

For the dtpa group 70 females were studied (mean age 10.9 years) and 49 males were studied (mean age 10.8 years). For the dT group 62 females were studed (mean age 10.9 years) and 56 males were studied (mean age 11.0 years).

The subjects included in the trial had received up to 4 doses of DTPw within the first 2 years of life.

Subjects excluded from the study were those who had had D, T or P vaccination after the second year of life or a history of diphtheria, tetanus or pertussis disease.

Sera were obtained pre and 1 month post vaccination with a solicited follow-up after 15 days and an unsolicited follow-up after 30 days.

Reactogenicity data, antibody responses, anti-PT results, anti-FHA results and anti-PRN (pertactin) results are shown in the appended figures.

It may be seen from the results that a dtpa vaccine according to the invention was safe and immunogenic in adolescents.

Note: In the figures, results relating to the antibody responses for the study in adults (dtpa 005) and the study in adolescents (dtpa 003) show the distribution of individual pre- and post-vaccination anti-diphtheria and anti-tetanus antibody titres of subjects included in the overall analysis of immunogenicity (>=0.1 IU/ml being the cutoff determined for protection). Also given are the geometric mean anti-diphtheria and anti-tetanus antibody titres in IU/ml.

The figures for the dtpa 005 and dtpa 003 studies which illustrate anti-PT, anti-FHA and anti-PRN titers show the vaccine response rate (in % of the total number of subjects - n -) and geometric mean antibody titres (GMT, in EU/ml) for the vaccine components PT, FHA and PRN of subjects included in the overall analysis of immunogenicity.

### Example 3: Preparation of a Hep B - dtpa vaccine

The formulation was prepared exactly as in Example 5 of WO93/24148 except that low doses of D, T, PT, FHA and 69K as hereinabove defined were used to make up 0.5 ml dose of bulk vaccine.

In a preferred composition the amounts of dtpa were approximately as follows: PT (8µg), FHA (8µg), 69K (2.5µg), D (2 Lf) and T (5 Lf) per 0.5 ml dose of bulk vaccine.

In another composition the amount of D was increased to 2.5Lf.

The amount of hepatitis B surface antigen in the vaccine composition can vary from 5-20 µg and is typically 10µg per 0.5 ml dose of bulk vaccine.

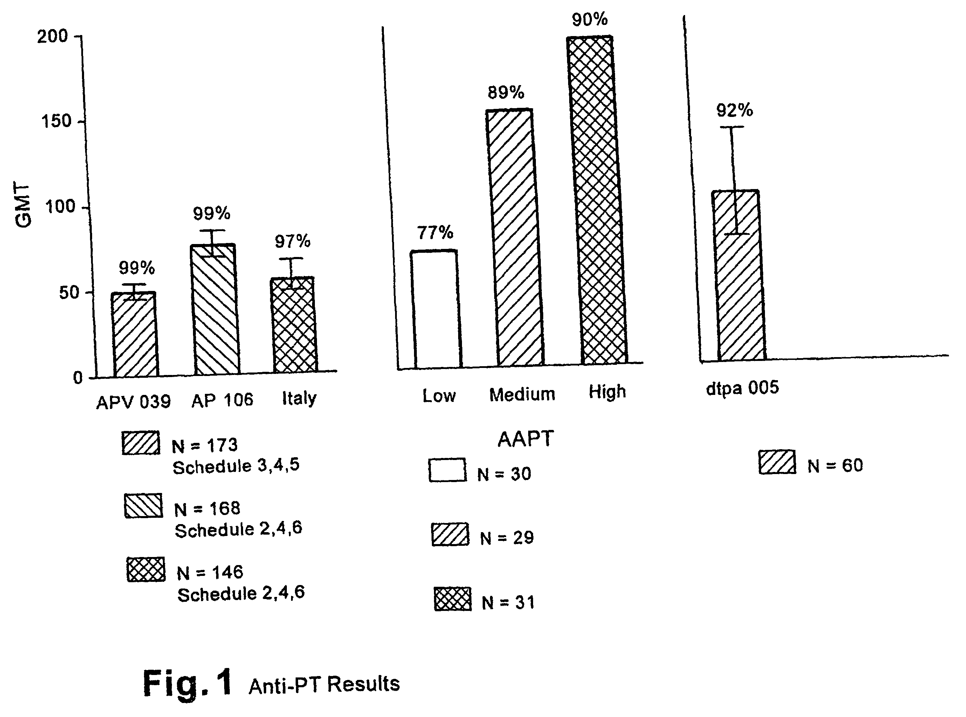

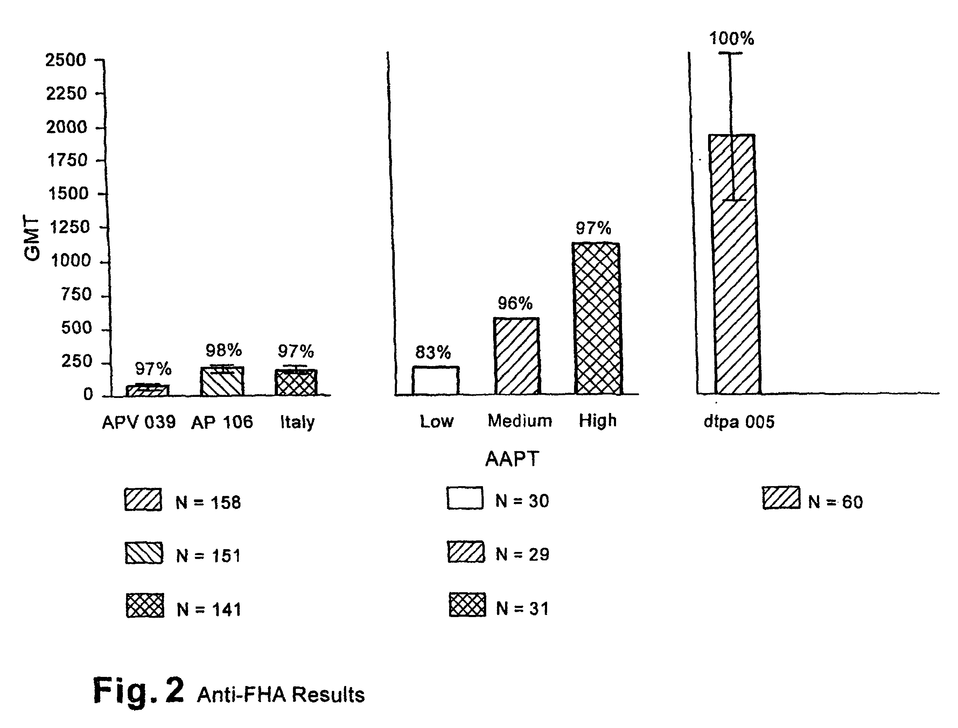

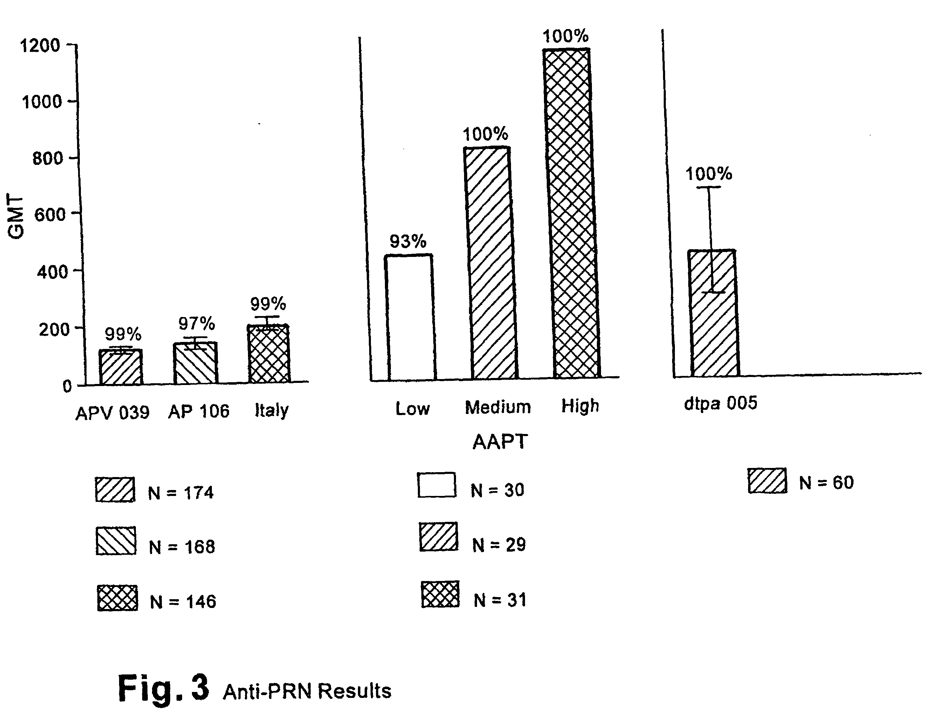

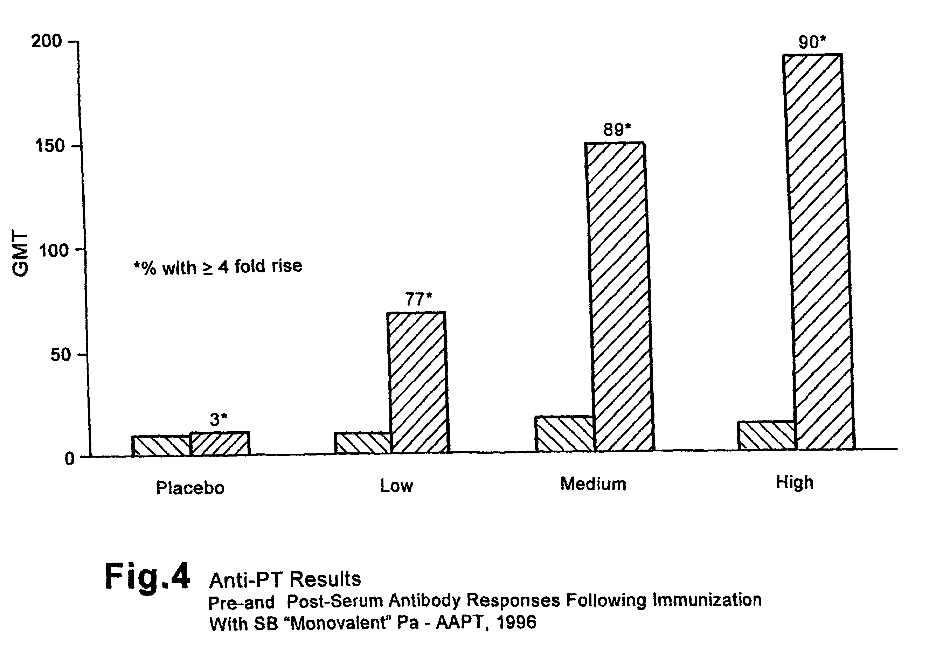

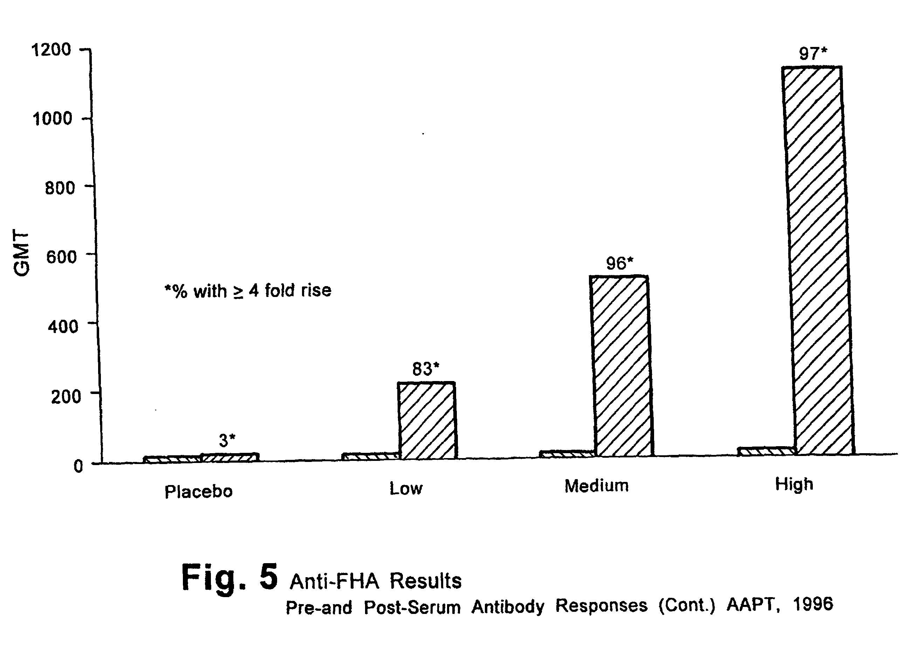

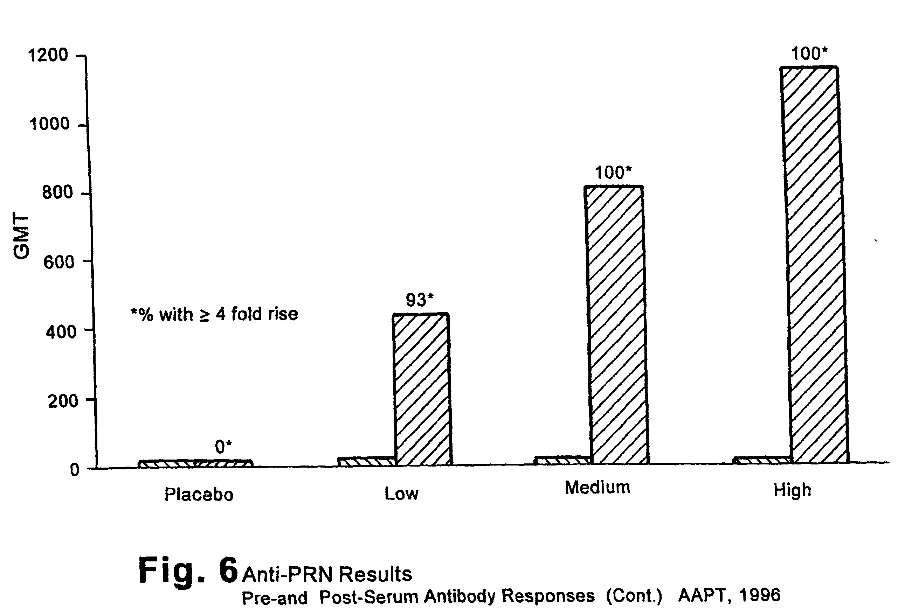

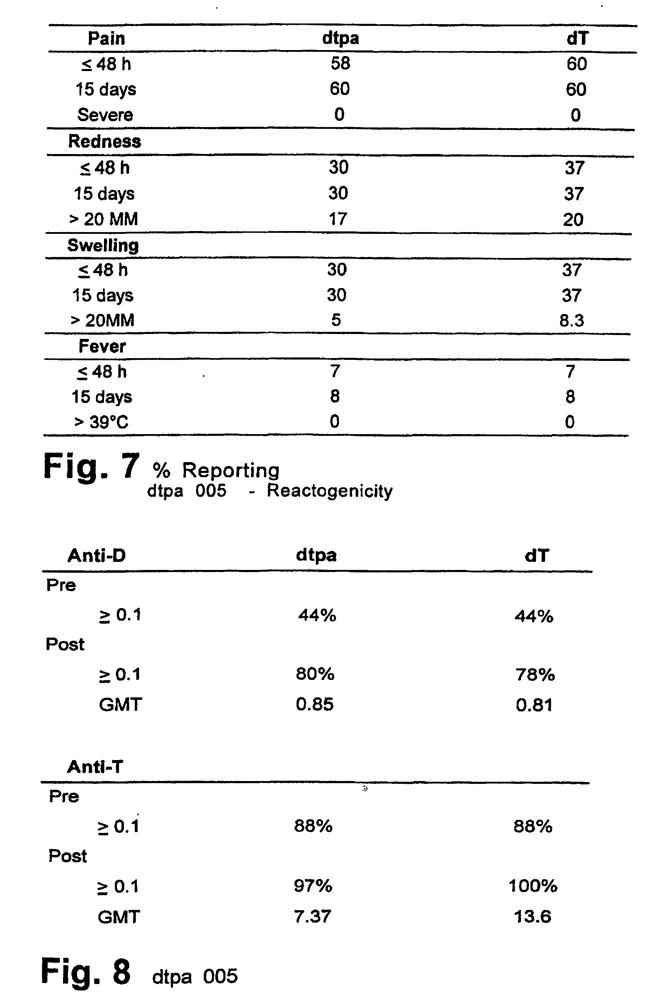

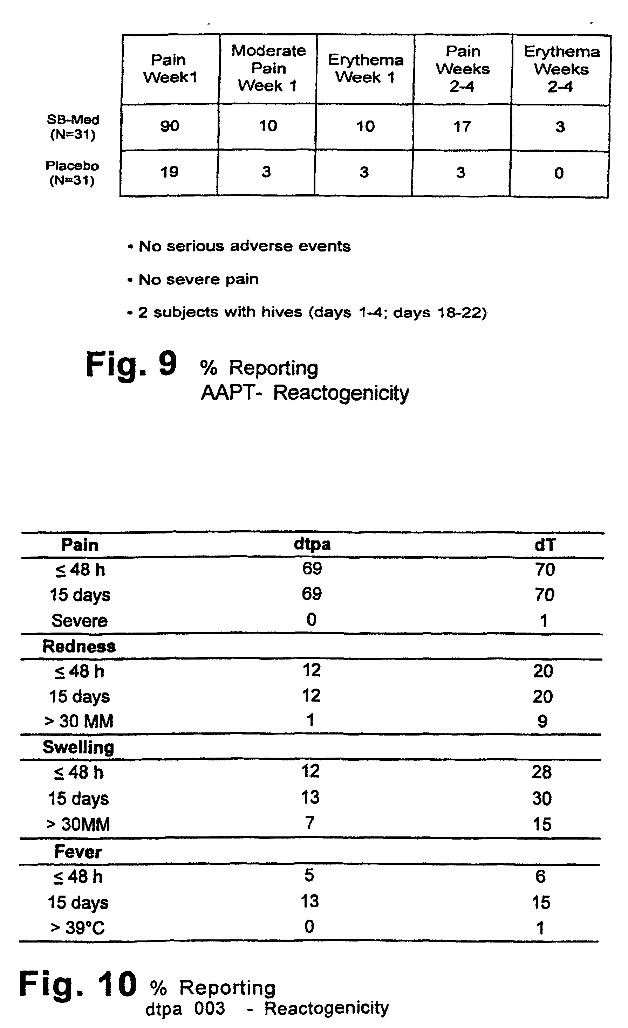

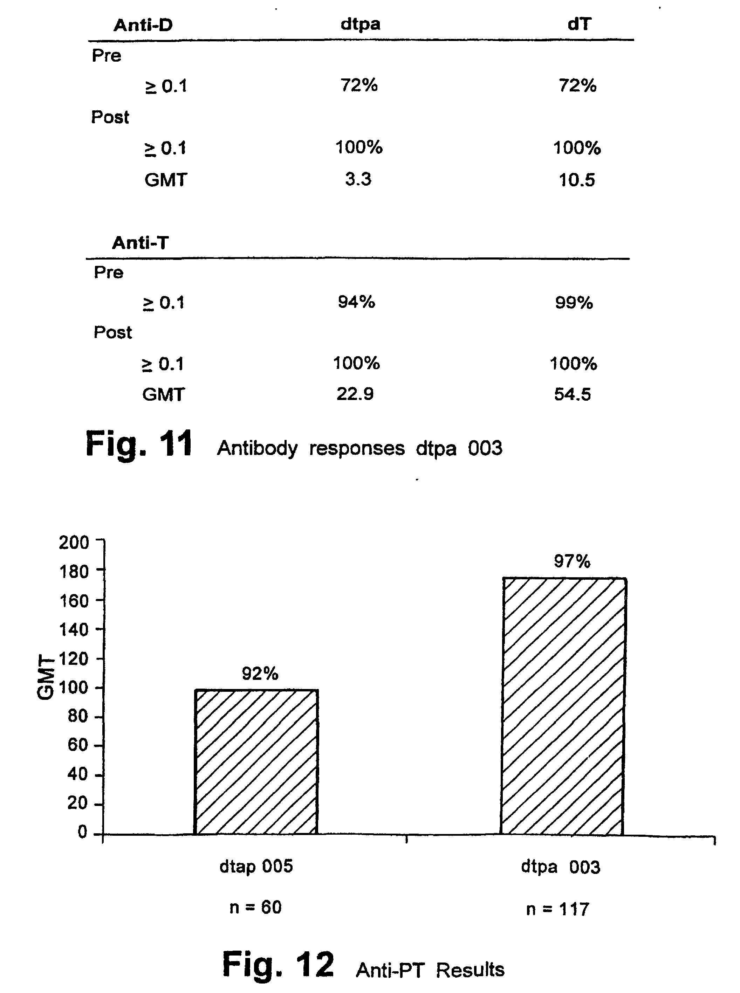

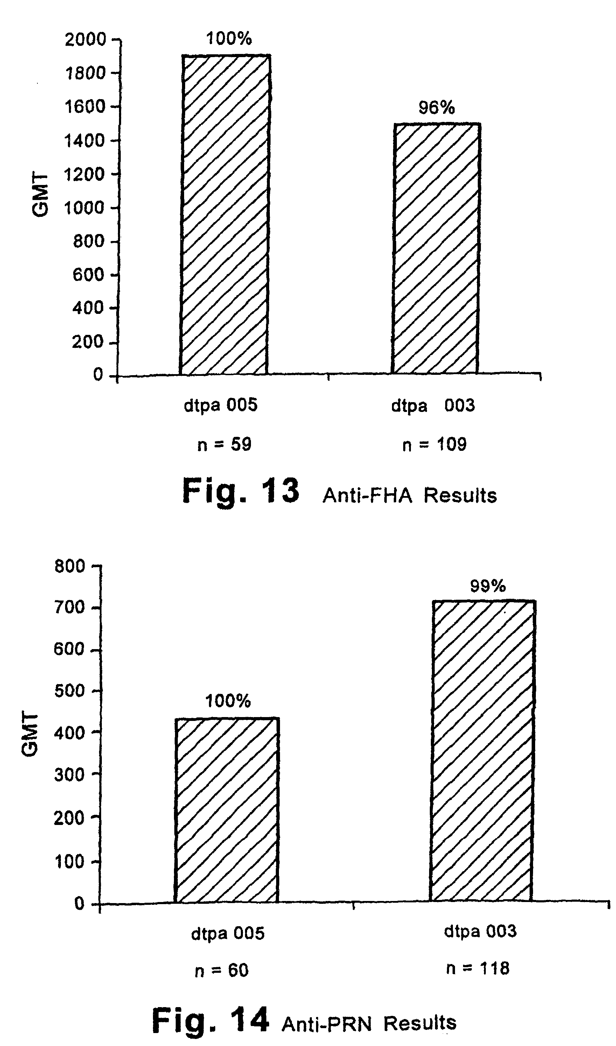

## Claims

1. A vaccine composition comprising diphtheria (D), tetanus (T) and acellular pertussis (Pa) antigens and an adjuvant, wherein the concentration of D per 0.5 ml dose of bulk vaccine is 1-4Lf; the concentration of T per 0.5 ml dose of bulk vaccine is 2.5 - 7.5 Lf and the Pa component comprises PT (pertussis toxoid), FHA (filamentous hemagglutinin) and pertactin (69K) wherein the concentration of PT per 0.5 ml dose of bulk vaccine is 2-10µg; the concentration of FHA per 0.5 ml dose of bulk vaccine is 2-10µg; and the concentration of 69K is in the range 0.5µg to 3µg per 0.5 ml dose of bulk vaccine.

2. A vaccine composition according to claim 1 having the composition: PT (8µg), FHA (8µg), 69K (2.5µg), D (2 Lf) and T (5 Lf) per 0.5 ml dose of bulk vaccine.

3. A vaccine composition according to any one of claims 1 or 2 which additionally comprises one or more additional antigens.

4. A vaccine composition according to claim 3 wherein an additional antigen is hepatitis B surface antigen.

5. A vaccine composition according to claim 4 wherein the hepatitis B surface antigen is the S-antigen of HBsAg.

6. A vaccine composition according to claim 3 or claim 4 wherein an additional antigen is present which provides immunity against one or more of Hib, polio or hepatitis A infection.

7. A vaccine composition according to any previous claim wherein the adjuvant used in the formulation comprises aluminium hydroxide.

8. A vaccine composition according to any previous claim wherein the adjuvant used in the formulation comprises aluminium phosphate.

9. Use of the vaccine composition according to any one of claims 1 to 8 in the manufacture of a medicament for use in preventing infection of Bordetella pertussis in children or adolescent or adult subjects.

## Patentansprüche

1. Impfstoffzusammensetzung, umfassend Diphtherie (D)-, Tetanus (T)- und azelluläre Pertussis (Pa)-Antigene und ein Adjuvans, wobei die Konzentration von D pro 0,5 ml-Dosis Impfstoffmenge 1-4 Lf, die Konzentration von T pro 0,5 ml-Dosis Impfstoffmenge 2,5-7,5 Lf beträgt und der Pa-Bestandteil PT (Pertussistoxoid), FHA (filamentöses Hämagglutinin) und Pertactin (69K) umfasst, wobei die Konzentration von PT pro 0,5 ml-Dosis Impfstoffmenge 2-10 µg, die Konzentration von FHA pro 0,5 ml-Dosis Impfstoffmenge 2-10 µg beträgt und die Konzentration von 69 K im Bereich von 0,5 µg bis 3 µg pro 0,5 ml-Dosis Impfstoffmenge liegt.

2. Impfstoffzusammensetzung nach Anspruch 1 mit der folgenden Zusammensetzung: PT (8 µg), FHA (8 µg), 69 K (2,5 µg), D (2 Lf) und T (5 Lf) pro 0,5 ml-Dosis Impfstoffmenge.

3. Impfstoffzusammensetzung nach Anspruch 1 oder 2, die zusätzlich ein oder mehrere zusätzliche Antigene umfasst.

4. Impfstoffzusammensetzung nach Anspruch 3, wobei ein zusätzliches Antigen das Hepatitls-B-Oberflächenantigen ist.

5. Impfstoffzusammensetzung nach Anspruch 4, wobei das Hepatitis-B-Oberflächenantigen das S-Antigen von HBsAg ist.

6. Impfstoffzusammensetzung nach Anspruch 3 oder 4, wobei ein zusätzliches Antigen vorhanden ist, das Immunität gegen eine oder mehrere der folgenden Infektionen: Hib, Polio oder Hepatitis-A verleiht.

7. Impfstoffzusammensetzung nach einem der vorangehenden Ansprüche, wobei das in der Formulierung verwendete Adjuvans Aluminiumhydroxid umfasst.

8. Impfstoffzusammensetzung nach einem der vorangehenden Ansprüche, wobei das in der Formulierung verwendete Adjuvans Aluminiumphosphat umfasst.

9. Verwendung der Impfstoffzusammensetzung nach einem der Ansprüche 1 bis 8 für die Herstellung eines Arzneimittels zur Verwendung bei der Vorbeugung einer Bordetella-Pertussis-Infektion bei Kindern oder Jugendlichen oder Erwachsenen.

## Revendications

1. Composition vaccinale comprenant des antigènes diphtériques (D), tétaniques (T) et coquelucheux acellulaires (Pa) ainsi qu'un adjuvant, dans laquelle la concentration en D par dose de 0,5 ml de vaccin en vrac est de 1 à 4 Lf ; la concentration en T par dose de 0,5 ml de vaccin en vrac est de 2,5 à 7,5 Lf et le composant Pa comprend de la PT (anatoxine pertussique), de la FHA (hémagglutinine filamenteuse) et de la pertactine (69K), la concentration en PT par dose de 0,5 ml de vaccin en vrac étant de 2 à 10 µg ; la concentration en FHA par dose de 0,5 ml de vaccin en vrac étant de 2 à 10 µg ; et la concentration en 69K étant dans la plage de 0,5 µg à 3 µg par dose de 0,5 ml de vaccin en vrac.

2. Composition vaccinale selon la revendication ayant la composition : PT (8 µg), FHA (8 µg), 69K (2,5 µg), D (2 Lf) et T (5 Lf) par dose de 0,5 ml de vaccin en vrac.

3. Composition vaccinale selon l'une quelconque des revendications 1 ou 2, laquelle comprend en outre un ou plusieurs antigènes supplémentaires.

4. Composition vaccinale selon la revendication 3, dans laquelle un antigène supplémentaire est l'antigène de surface de l'hépatite B.

5. Composition vaccinale selon la revendication 4, dans laquelle l'antigène de surface de l'hépatite B est l'antigène S de HBsAg.

6. Composition vaccinale selon la revendication 3 ou la revendication 4, dans laquelle un antigène supplémentaire est présent, lequel confère une immunité contre une ou plusieurs des infections comprenant le Hib, la polio et l'hépatite A.

7. Composition vaccinale selon l'une quelconque des revendications précédentes, dans laquelle l'adjuvant utilisé dans la formulation comprend de l'hydroxyde d'aluminium.

8. Composition vaccinale selon l'une quelconque des revendications précédentes, dans laquelle l'adjuvant utilisé dans la formulation comprend du phosphate d'aluminium.

9. Utilisation de la composition vaccinale selon l'une quelconque des revendications 1 à 8 dans la fabrication d'un médicament destiné à prévenir une infection par Bordetella pertussis chez des sujets enfants, adolescents ou adultes.
